(19) **Europäisches Patentamt / European Patent Office / Office européen des brevets**

(11) **EP 3 291 733 B1**

(12) **FASCICULE DE BREVET EUROPEEN**

(45) Date de publication et mention de la délivrance du brevet:
**10.04.2019 Bulletin 2019/15**

(21) Numéro de dépôt: **16720837.0**

(22) Date de dépôt: **04.05.2016**

(51) Int Cl.:
*A61B 6/02* (2006.01)      *A61B 6/00* (2006.01)

(86) Numéro de dépôt international:
**PCT/EP2016/060035**

(87) Numéro de publication internationale:
**WO 2016/177798 (10.11.2016 Gazette 2016/45)**

(54) **ESTIMATION DE LA REPARTITION DE LA DENSITE MINERALE OSSEUSE DANS AU MOINS UNE PARTIE DE SQUELETTE D'UN INDIVIDU**

SCHÄTZUNG DER VERTEILUNG DER KNOCHENMINERALDICHTE IN MINDESTENS EINEM TEIL DES SKELETTS EINER PERSON

ESTIMATING THE DISTRIBUTION OF BONE MINERAL DENSITY IN AT LEAST ONE PORTION OF A PERSON'S SKELETON

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.05.2015 FR 1554150**

(43) Date de publication de la demande:
**14.03.2018 Bulletin 2018/11**

(73) Titulaires:
- **AMVALOR**
  **75013 Paris (FR)**
- **ECOLE NATIONALE SUPERIEURE D'ARTS ET METIERS**
  **75013 Paris (FR)**

(72) Inventeur: **SKALLI, Wafa**
**75013 Paris (FR)**

(74) Mandataire: **Lavialle, Bruno François Stéphane et al**
**Cabinet Boettcher**
**16, rue Médéric**
**75017 Paris (FR)**

(56) Documents cités:
**US-A1- 2001 004 394      US-A1- 2002 075 997**
**US-A1- 2006 204 069      US-A1- 2011 058 725**

**Description**

L'invention concerne le domaine des procédés d'estimation de la répartition de la densité minérale osseuse dans au moins une partie de squelette d'un individu.

ARRIERE PLAN DE L'INVENTION

Il est connu des procédés pour caractériser la répartition de la densité minérale osseuse dans le squelette d'individus. Cette caractérisation peut être particulièrement utile pour diagnostiquer un risque d'ostéoporose d'un individu.

A cet effet on utilise des images radiologiques du squelette de l'individu réalisées en projetant depuis une source de rayons X, tel qu'un tube à rayons X, des rayons X à différents niveaux d'énergie sur l'individu et en détectant, pour chacun de ces niveaux d'énergie, les atténuations de ces rayons X par les tissus de l'individu.

L'analyse de l'atténuation différentielle entre les tissus permet de visualiser sur un plan de projection du squelette de l'individu, une répartition surfacique de la densité minérale osseuse.

Un inconvénient de ce procédé connu sous le nom de « Dual X-Ray absortiometry » pour absorptiométrie bi photonique à rayons X, est que lorsque l'appareil radiologique nécessite un temps entre la génération des deux niveaux d'énergie, l'individu doit rester immobile, sans quoi on ne peut pas réaliser d'analyse différentielle entre les deux images obtenues aux deux niveaux d'énergie.

Un autre problème de cette méthode est qu'il nécessite un niveau de formation important pour les opérateurs de radiologie ainsi que des installations spécifiques à la génération de rayons X bi énergie.

Le document US2006/0204069A1 divulgue une procédé connue pour calculer la densité minérale osseuse du corps osseux.

OBJET DE L'INVENTION

Un objet de la présente invention est de fournir un procédé alternatif d'estimation de la répartition de la densité minérale osseuse dans au moins une partie de squelette d'un individu.

RESUME DE L'INVENTION

Afin de répondre à cet objectif, il est proposé selon l'invention un procédé d'estimation de la répartition de la densité minérale osseuse dans au moins une partie de squelette d'un individu, ce squelette étant composé de tissus osseux, ces tissus osseux étant entourés de tissus mous eux même entourés par une enveloppe externe de l'individu.

Ce procédé est essentiellement caractérisé en ce qu'il comprend la génération :

- d'une représentation géométrique en trois dimensions de ladite partie du squelette de l'individu; et
- d'une représentation géométrique en trois dimensions d'au moins une portion de l'enveloppe externe contenant ladite partie du squelette de l'individu ;
- d'au moins une première image radiologique de ladite au moins une partie du squelette, cette première image étant générée par observation d'une projection de rayons X, provenant d'une première source, sur une première surface de détection, cette première image étant générée par projection de rayons X et représentant l'individu observé suivant une première incidence d'observation ;
- d'au moins une deuxième image radiologique de ladite au moins une partie de squelette, cette deuxième image étant générée par projection de rayons X et représentant l'individu observé suivant une deuxième incidence d'observation distincte de la première incidence d'observation; puis
à l'aide d'un logiciel prédéterminé, et pour plusieurs rayons X donnés parmi lesdits rayons X projetés, on estime pour chaque rayon X donné :

- une valeur d'épaisseur de tissus mous traversés par ce rayon X donné ;
- une valeur d'épaisseur de tissus osseux traversés par ce rayon X donné ; et
- une valeur d'atténuation globale de ce rayon X donné, entre sa source et sa projection sur la surface de détection où il est projeté ; et

• à l'aide de la valeur d'épaisseur de tissus mous traversés par ce rayon X donné, et d'une estimée d'un coefficient d'atténuation des tissus mous traversés par ce rayon X donné, on déduit d'une part une valeur d'atténuation de ce rayon X donné par l'épaisseur de tissus mous qu'il a traversée et d'autre part une valeur d'atténuation de ce rayon X donné par l'épaisseur de tissus osseux qu'il a traversée ; puis
• à l'aide de la valeur d'épaisseur de tissus osseux traversée par ce rayon X donné, de la valeur d'atténuation de ce rayon X donné par l'épaisseur de tissus osseux qu'il a traversée, et de la valeur d'atténuation globale de ce rayon X donné, le logiciel estime une valeur représentative de la densité minérale osseuse des tissus osseux traversés par ce rayon X donné.

Pour la compréhension de l'invention, le terme rayon X correspond à au moins un rayon X ou plusieurs rayon X parallèles entre eux et formant un faisceau.

Toujours pour la compréhension de l'invention, le terme coefficient d'atténuation désigne un coefficient permettant de quantifier l'atténuation de l'intensité d'un rayon X donné par une matière donnée en fonction de l'épaisseur de cette matière traversée par ce rayon X.

Chaque rayon X projeté depuis une source de projection se propage dans l'espace et jusqu'à la surface de détection qui lui correspond en suivant une trajectoire rectiligne. Ainsi chaque rayon projeté traverse les tissus mous et les tissus osseux du squelette de l'individu en suivant une ligne droite. Connaissant la source du rayon et son point de projection, c'est-à-dire son point d'impact sur la surface de détection, on peut déterminer la position dans l'espace de chaque rayon X par rapport aux surfaces de détection et par rapport aux sources, sachant que ces surfaces de détection et sources sont fixes les unes par rapport aux autres au moins pendant le temps nécessaire à la projection et que les positions relatives entre ces surfaces de détection et les sources sont connues, notamment par calibration de l'environnement radiographique.

Sachant que chaque rayon X traversant une matière est atténué par cette matière en fonction d'un coefficient d'atténuation propre à cette matière, on constate que chaque rayon X traversant l'enveloppe de l'individu, les tissus mous et/ou tissus osseux de l'individu est atténué par l'ensemble des tissus qu'il a traversé.

Comme chaque point de l'image radiologique présente un niveau de gris ou de couleur ou de contraste qui est représentatif / fonction de l'intensité du rayon X arrivant en un point correspondant de la surface de détection, on peut déterminer par observation de chaque point de l'image radiologique, un niveau d'intensité du ou des rayons X qui ont été projetés sur le point correspondant de la surface de détection correspondante.

Connaissant l'intensité de chaque rayon X émis par la source donnée qui lui correspond et connaissant son niveau d'intensité à son arrivée sur la surface de détection correspondante, on peut retrouver à partir de chaque point d'une image radiologique, un niveau d'atténuation subie par le ou les rayons X qui sont arrivés / ont été projetés sur ce point depuis la source correspondante.

Ainsi, par contraste entre les points de l'image, on obtient les limites projetées des différents tissus mous, des différents tissus osseux et de l'enveloppe de l'individu.

Comme les sources des rayons et les surfaces de détection sont fixes dans l'espace et localisées dans l'espace à l'aide d'un repère spatial et comme l'individu conserve la même posture pendant le temps de la projection, on peut en observant les première et deuxième images radiologiques, déduire aux incertitudes quantifiables près, les positions exactes dans ledit référentiel spatial de chaque point de l'individu qui a été traversé :

- d'une part par un rayon X ou faisceau de rayons X provenant de la première source et projeté(s) sur la première surface de détection comme illustré sur la première image radiologique; et

- d'autre part par un autre rayon X ou faisceau de rayons X utilisé pour générer la deuxième image radiologique.

On note que le changement d'incidence d'observation entre les première et deuxième images radiologiques peut être obtenu :

- soit en déplaçant, la source de rayons X par rapport à l'individu observé, tout en s'assurant qu'il conserve une même posture entre les prises de vues nécessaires à l'obtention des première et deuxième images ;
- soit en utilisant deux sources de rayons X distinctes et/ou deux surfaces de détection distinctes.

Ainsi, l'analyse des première et deuxième images radiologiques, réalisées selon des incidences d'observation de l'individu distinctes, c'est à dire différentes l'une de l'autre, permet :

- d'une part d'avoir une représentation spatiale de l'individu, de son enveloppe et de ses tissus mous et osseux ; et
- d'autre part d'avoir une estimation de l'atténuation globale subie par un rayon traversant plusieurs tissus mous et éventuellement plusieurs tissus osseux de l'individu.

Toutefois la seule estimation d'une valeur d'atténuation globale du rayon X donné, entre sa source et sa projection sur la surface de détection, n'est pas suffisante pour estimer la densité osseuse des tissus osseux traversés par le rayon X donné. En effet, une part de cette valeur d'atténuation globale du rayon X donné est liée non pas aux tissus osseux, mais à des tissus mous.

Le procédé selon l'invention apporte une solution à ce problème puisqu'il permet d'estimer la part d'atténuation d'un rayon X donné qui est liée aux seuls tissus mous traversés par ce rayon X donné et la part d'atténuation de ce rayon X donné qui est liée aux tissus osseux traversés par ce rayon X.

En effet, le procédé selon l'invention utilise :

- une représentation géométrique en trois dimensions de ladite partie du squelette de l'individu, et
- une représentation géométrique en trois dimensions d'au moins une portion de l'enveloppe externe contenant ladite partie du squelette de l'individu ; et

connaissant la position de chaque rayon X donné par rapport à ces représentations géométriques de la partie du squelette et de l'enveloppe externe, on sait calculer à l'aide du logiciel :

- d'une part la distance totale parcourue par ce rayon X donné au travers des tissus mous situés dans l'enveloppe de l'individu ; et
- d'autre part la distance totale parcourue par ce même rayon X donné au travers des tissus osseux situés dans l'enveloppe de l'individu.

Ces deux distances correspondent respectivement à :

- une valeur d'épaisseur de tissus mous traversés par ce rayon X donné ; et
- une valeur d'épaisseur de tissus osseux traversés par ce rayon X donné.

Il existe plusieurs moyens pour estimer le coefficient d'atténuation des tissus mous de l'individu.

On peut par exemple rechercher un rayon X particulier traversant uniquement des tissus mous de l'individu sans traverser de tissus osseux.

Pour estimer le coefficient d'atténuation des tissus mous de l'individu, le logiciel recherche un rayon X particulier traversant uniquement des tissus mous de l'individu sans traverser de tissus osseux et à partir de la position de ce rayon X particulier et de la représentation géométrique en trois dimensions de la portion de l'enveloppe externe, le logiciel estime la valeur d'épaisseur de tissus mous traversés par ce rayon X particulier, et par observation d'un point de projection de ce rayon x particulier, le logiciel détermine une valeur d'atténuation subie par ce rayon X particulier lors de sa traversée des seuls tissus mous de l'individu, puis connaissant ladite valeur d'épaisseur de tissus mous traversés par ce rayon X particulier et ladite valeur d'atténuation subie par ce rayon X particulier, le logiciel calcule le coefficient d'atténuation associé aux seuls tissus mous de l'individu.

Il s'agit ici d'un coefficient d'atténuation spécifique aux tissus mous de l'individu.

Il est bien entendu tout à fait possible, pour améliorer la précision, de calculer ce coefficient pour différents rayons X ne traversant que les tissus mous, dans une région considérée comme homogène, et d'estimer le coefficient d'atténuation comme une moyenne des différentes valeurs obtenues.

Puis, pour tout rayon X donné, connaissant :

- le coefficient d'atténuation des tissus mous de l'individu ; et
- l'épaisseur de tissus mous traversée par ce rayon X donné, le logiciel calcule :
- d'une part une valeur d'atténuation de ce rayon X donné par l'épaisseur de tissus mous qu'il a traversée ; et
- d'autre part, en utilisant une intensité I0 de ce rayon donné à sa source et une intensité I de ce rayon donné telle que détectée sur la surface de détection sur laquelle il est projeté, le logiciel calcule une valeur d'atténuation globale de ce rayon X donné, entre sa source et la surface de détection sur laquelle il est projeté ; puis
- en corrigeant la valeur d'atténuation globale de ce rayon X donné pour tenir compte de ladite valeur d'atténuation de ce rayon X donné par l'épaisseur de tissus mous qu'il a traversée, le logiciel calcule la valeur d'atténuation de ce rayon X donné par l'épaisseur de tissus osseux qu'il a traversée.

Ainsi, pour chaque rayon X donné, on peut calculer la valeur d'atténuation du rayon X donné par la seule épaisseur de tissus osseux qu'il a traversée.

On peut décider que ladite valeur représentative de la densité minérale osseuse des tissus osseux traversés par ce rayon X donné soit un coefficient d'atténuation du tissu osseux traversé par ce rayon x donné.

Comme on le verra en détail ci-après, connaissant d'une part l'épaisseur du tissus osseux traversé par ce rayon X donné et d'autre part le niveau d'atténuation de ce rayon X imputable à la seule traversée de ce tissus osseux, il est possible de calculer une densité minérale osseuse dite surfacique qui correspond aux tissus osseux qui ont été traversés par ce rayon X donné. Connaissant les coefficients d'atténuation des tissus osseux estimés avec les différents rayons traversant la structure osseuse d'intérêt, caractérisant l'image projetée de cette structure, il est possible de calculer la densité minérale osseuse surfacique.

Il est à noter qu'en complément de la méthode précitée de détermination du coefficient d'atténuation associé aux seuls tissus mous, on peut aussi utiliser une méthode nécessitant une éprouvette de calibration présentant plusieurs zones ayant chacune un coefficient d'atténuation propre connu.

Cette méthode consiste à placer une ou plusieurs éprouvettes qui présentent chacune au moins un coefficient d'atténuation connu, à côté de l'individu lors de la projection des rayons depuis les sources vers les surfaces de détection.

Puis, en étudiant le niveau de gris ou de couleur ou de contraste sur les première et seconde images radiologiques, on peut obtenir une correspondance entre chaque coefficient d'atténuation propre connu d'une éprouvette et un niveau gris ou de couleur ou de contraste correspondant sur l'image.

On peut ainsi simplement estimer les atténuations subies par chaque rayon X donné projeté sur une surfaces de détection donnée, en recherchant dans une zone de projection de l'éprouvette visible sur une des première ou deuxième images de radiologie, un niveau de gris, de couleur ou de contraste associée équivalent au niveau de gris, de couleur ou de contraste constaté en un point de l'image correspondant au point de contact entre ce rayon X donné et la surface de détection correspondante. On peut ainsi déterminer l'atténuation d'intensité subie par chaque rayon X donné après avoir traversé les tissus de l'individu.

En effet, deux points de l'image présentant une intensité similaire ont une même valeur de produit mu*épaisseur, mu étant le coefficient d'atténuation propre à la matière traversée par un rayon X et l'épaisseur étant l'épaisseur de cette matière traversée par le rayon. Ainsi, connaissant l'épaisseur du milieu traversé et l'épaisseur de l'éprouvette on peut estimer le mu des tissus mous traversés.

Par correction, on peut ensuite estimer le coefficient moyen du tissu dur traversé.

Dans un mode de réalisation préférentiel, afin d'obtenir les deux images avec les première et deuxième incidences différentes, on peut faire en sorte que ladite deuxième image radiologique de ladite au moins une partie de squelette soit générée par observation d'une projection de rayons X, provenant d'une deuxième source distincte et éloignée de la première source, sur une deuxième surface de détection également distincte et éloignée de la première surface de détection.

Dans un mode particulier de réalisation de l'invention, ladite représentation géométrique en trois dimensions de ladite au moins une portion de l'enveloppe externe contenant ladite partie du squelette de l'individu est déduite à l'aide d'au moins une observation de l'enveloppe réalisée à l'aide de moyens de capture optique.

Idéalement, la représentation géométrique en trois dimensions de l'enveloppe de l'individu ainsi que les première et deuxième images radiologiques sont respectivement positionnées dans un même référentiel spatial et correspondent préférentiellement à un même instant d'observation de l'individu, ce qui permet de faciliter les calculs nécessaires à la mesure de répartition de densité minérale osseuse dans la partie observée du squelette.

Enfin, il est à noter que dans certains cas on peut générer la représentation géométrique en trois dimensions de l'enveloppe de l'individu en utilisant les première et deuxième images radiologiques.

Pour cela, le logiciel identifie sur les première et deuxième images radiologiques, des paramètres caractéristiques de l'individu tels que positions spatiales relatives entre des points caractéristiques du squelette et/ou de l'enveloppe externe de l'individu visible sur ces première et deuxième images, ou des dimensions ou des distances entre points caractéristiques du squelette et/ou de l'enveloppe externe visibles sur ces première et deuxième images.

Puis, le logiciel génère une représentation géométrique en trois dimensions de l'enveloppe de l'individu, à l'aide des paramètres caractéristiques de l'individu ainsi identifiés et à l'aide d'une base de données statistique préalablement élaborée avec une population d'individus, cette base de données illustrant des relations entre des paramètres caractéristiques d'individus et des représentations escomptées en trois dimensions de l'enveloppe de l'individu.

BREVE DESCRIPTION DES DESSINS

D'autres caractéristiques et avantages de l'invention ressortiront clairement de la description qui en est faite ci-après, à titre indicatif et nullement limitatif, en référence aux dessins annexés, dans lesquels:

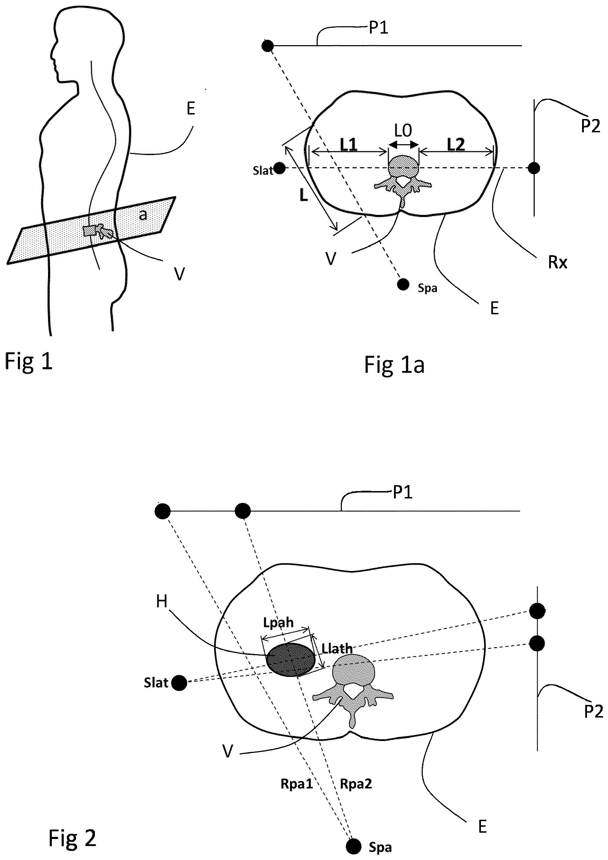

- La figure 1 présente une vue latérale d'un individu sur laquelle on voit l'enveloppe E de l'individu et un plan d'observation « a » choisi pour observer une partie du squelette de l'individu et de l'enveloppe, ici en coupe transversale;
- La figure 1a présente la vue en coupe transversale de la partie de squelette de l'individu selon le plan d'observation « a », dans le cas où les tissus mous de l'individu sont considérés comme relativement homogènes du point de vu de leurs coefficients d'atténuation, on voit sur cette figure des première et deuxième sources Slat et Spa de projection de rayons X et des première et deuxième surfaces planes P1, P2 (plans de détection) de détection de rayons X, chaque surface de détection présentant une pluralité de points de détection chacun adapté à détecter une intensité de rayons X projeté sur le point de détection ;
- La figure 2 illustre la même vue que celle de la figure 1a mais ici l'individu présente une hétérogénéité H de tissus mous conduisant à la prise en compte de plusieurs coefficients d'atténuation, en l'occurrence un coefficient devant être déterminé pour chaque hétérogénéité de tissus mous pour pouvoir estimer la répartition de la densité minérale osseuse en limitant le bruit généré par les tissus mous qui entourent les os et les hétérogénéités de ces tissus mous.

DESCRIPTION DETAILLEE DE L'INVENTION

Comme indiqué précédemment, l'invention porte sur un procédé d'estimation de la répartition de la densité minérale osseuse dans au moins une partie V de squelette d'un individu.

L'invention porte aussi sur un dispositif pour la mise en oeuvre de ce procédé. Comme on le voit sur les figures 1a et 2, ce dispositif peut comporter des première et deuxième surfaces de détection P1, P2 qui sont respectivement planes et sont orientées entre elles d'un angle de 90° avec une incertitude d'angle de plus ou moins 60° et préférentiellement de plus ou moins 5° d'angle et préférentiellement inférieure à 1° d'angle, comme sur les figures 1a et 2.

Ce dispositif comporte également :

- une première source de rayons X, dite source postéro antérieure Spa, pour projeter des rayons vers et sur la première surface de détection P1 en traversant l'individu en allant de la face postérieure de l'individu vers sa face antérieure ; et
- une deuxième source de rayons X, dite source latérale Slat, pour projeter des rayons vers et sur la deuxième surface de détection P2 en traversant l'individu entre ses faces latérales.

Le dispositif comporte aussi un calculateur et un logiciel permettant de réaliser des calculs ou des estimations ou déductions nécessaires à la mise en oeuvre du procédé selon l'invention, ce dispositif étant ainsi automatisé ou du moins semi automatisé pour permettre une estimation de la répartition surfacique ou volumique de densité minérale osseuse, ci-après DMO, dans au moins une partie du squelette d'un individu.

A noter que le dispositif peut être constitué d'une source mobile et de moyens de calibration pour connaitre dans chacune des vues l'environnement radiologique global (position des sources par rapport aux films et position relatives des sources), et si nécessaire d'un repérage sur le sujet permettant de positionner un sujet dans un environnement global.

Le dispositif peut aussi être constitué d'une seule source et d'un dispositif de rotation et de calibration permettant de tourner le sujet et de définir la paire d'images en prenant en compte la rotation du sujet pour avoir les deux incidences différentes.

Le dispositif selon l'invention peut aussi comporter des moyens de capture optique d'observation de l'enveloppe externe E de l'individu. Ces moyens de capture optique qui sont reliés au calculateur permettent de générer une représentation géométrique en trois dimensions d'au moins une portion de l'enveloppe externe E contenant ladite partie du squelette de l'individu.

Ces moyens de capture optique, non représentés, peuvent être un scanner de corps ou une caméra ou appareil photo, stéréoscopique ou non.

Ces moyens de capture optique peuvent éventuellement être associés à des moyens de projection de franges de moiré ou de trame éventuellement générée via des projections de lumières sur l'enveloppe de l'individu. Ces moyens de projection peuvent être par exemple adaptés à projeter les franges ou trames sous forme de lumière laser.

Pour faciliter l'analyse des images obtenues par le dispositif de l'invention, les positions des première et deuxième surfaces de détection et les positions des première et deuxième sources de rayons X ainsi qu'éventuellement celles des moyens de capture optique sont localisées dans un même espace à l'aide d'un repère spatial.

L'objectif du procédé selon l'invention est de permettre la fourniture d'une estimée de la répartition minérale osseuse DMO dans une partie de squelette d'un individu.

La partie, plus particulièrement ciblée du squelette pour l'estimation de la répartition de DMO est essentiellement un corps vertébral ou une vertèbre, ou un col du fémur ou plus généralement le fémur. Ces parties sont ciblées car elles sont particulièrement adaptées à la caractérisation du risque d'ostéoporose ou d'une évolution de densité minérale.

Dans un premier temps, pour la mise en oeuvre du procédé selon l'invention, on génère :

- une représentation géométrique en trois dimensions de ladite partie du squelette V de l'individu dont on veut étudier la répartition de DMO; et
- une représentation géométrique en trois dimensions d'au moins une portion de l'enveloppe externe E contenant ladite partie du squelette V de l'individu ; et
- au moins une première image radiologique de ladite au moins une partie du squelette, cette première image étant générée par observation d'une projection de rayons X, provenant de la première source Spa, sur la première surface de détection P1; et
- au moins une deuxième image radiologique de ladite au moins une partie de squelette, cette deuxième image étant générée par observation d'une projection de rayons X, provenant d'une deuxième source Slat distincte et éloignée de la première source, sur une deuxième surface de détection.

Idéalement, ces première et deuxième images correspondent respectivement à un même instant de détection des rayons X projetés sur les première et deuxième surfaces de détection. Par même instant, on entend que l'on a un décalage temporel entre les détections nécessaires à la génération des première et deuxième images qui est inférieur à 1 seconde et préférentiellement inférieur à 0.5 seconde.

Idéalement, comme il s'agit d'obtenir une répartition de DMO sur le squelette en vue, par exemple d'une analyse de risque d'ostéoporose, les première et deuxième images sont réalisées pour illustrer des projections de l'individu en position debout. La position debout de l'individu est la plus représentative des risques associés à l'ostéoporose.

Bien qu'il soit possible d'obtenir ladite représentation géométrique en trois dimensions de ladite au moins une portion de l'enveloppe externe contenant ladite partie du squelette de l'individu à partir d'une observation de l'individu réalisée avec les moyens de capture optique, il est aussi possible que cette représentation géométrique en trois dimensions soit déduite à partir desdites première et deuxième images radiologiques.

Dans certains cas, il est possible de combiner ces deux techniques, les moyens de capture optique permettant de s'assurer de la précision de représentation de l'enveloppe externe et l'usage des première et deuxième images permettant d'assurer que la représentation de l'enveloppe soit bien positionnée dans le même référentiel spatial que la représentation en trois dimensions de la partie du squelette que l'on veut visualiser.

Dans le cas où les moyens de capture optique ne fourniraient qu'une seule vue de l'enveloppe externe et en considérant un modèle 3D de vertèbre à partir de la seule image radiographique estimée, il est alors possible d'estimer les épaisseurs de tissus mous traversés et ainsi évaluer l'atténuation des rayons X par ces tissus mous. La DMO surfacique de la vertèbre peut ainsi être estimée mais avec une précision réduite.

On note qu'il existe plusieurs solutions pour déterminer une représentation géométrique en trois dimensions de ladite partie du squelette de l'individu.

On notera par exemple les solutions illustrées dans les documents brevets FR2856170 et WO2008146069.

La publication Humbert L, De Guise JA, Aubert B, Godbout B, Skalli W: 3D reconstruction of the spine from biplanar X-rays using parametric models based on transversal and longitudinal inferences. Med Eng Phys. 2009 Jul;31(6):681-7 décrit un exemple de méthodologie pour reconstruire un modèle virtuel d'une partie de squelette d'un individu à l'aide d'une radiographie biplane, en générant des images du type desdites première et deuxième images radiologiques.

Les deux publications suivantes traitent à la fois de la reconstruction 3D du squelette et de celle de l'enveloppe externe.

La publication "Personalized Body Segment Parameters From Biplanar Low-Dose Radiography" de Raphael Dumas, Rachid Aissaoui, Member, IEEE, David Mitton, Wafa Skalli, et Jacques A. de Guise publiée au journal IEEE TRANSACTIONS ON BIOMEDICAL ENGINEERING, Vol 52, NO. 10 Octobre 2005, décrit aussi une méthodologie pour reconstruire un modèle virtuel d'une partie de squelette d'un individu à l'aide d'une radiographie biplane, en générant des images du type desdites première et deuxième images radiologiques. Cette méthodologie donne un exemple d'obtention d'un modèle virtuel représentatif d'une partie de squelette / représentation géométrique en trois dimensions de ladite partie du squelette de l'individu. Ce type de modèle obtenu conformément à la méthode décrite dans cette publication est utilisable pour la mise en oeuvre de la présente invention.

De même, la publication "Subject-specific body segment parameters estimation using biplanar X-rays: a feasibility study" de Baptiste Sandoz, Sébastien Laporte, Wafa Skalli and David Mitton CNRS, Arts et Métiers Paris Tech, LBM, 151 bd de l'Hôpital 75013 Paris, France, publiée au journal Computer Methods in Biomechanics and Biomédical Engineering Vol. 13, No. 6, December 2010, 649-654, par l'éditeur Taylor & Francis, décrit également une méthode permettant, à partir d'images de radiologie du type desdites première et deuxième images générées dans des plans distincts non parallèles, d'obtenir un modèle virtuel représentatif d'une partie de squelette et de l'enveloppe externe.

Ce type de modèle obtenu conformément à la méthode décrite dans cette dernière publication est utilisable pour la mise en oeuvre de la présente invention.

Après avoir déterminé :

- la représentation géométrique en trois dimensions de ladite partie du squelette de l'individu; et
- la représentation géométrique en trois dimensions de l'enveloppe externe E ;

Le logiciel estime automatiquement et pour chaque rayon X Rx donné :

- une valeur d'épaisseur de tissus mous $L_1$, $L_2$ traversés par ce rayon X donné Rx ;
- une valeur d'épaisseur de tissus osseux $L_0$ traversés par ce rayon X donné Rx ; et
- une valeur d'atténuation globale de ce rayon X donné, entre sa source et sa projection sur la surface de détection où il est projeté.

La valeur d'atténuation d'un rayon X donné pour un tissu traversé d'épaisseur x se calcule à partir de l'intensité $I_0$ du rayon X (aussi appelé faisceau incident) en sortie de sa source Slat ou Spa et de l'intensité I de ce même rayon X en sortie du tissu traversé, selon la formule $I = I_0\,e^{-\mu x}$, $\mu$ étant le coefficient d'atténuation du tissu

La valeur d'atténuation globale d'un rayon X donné traversant plusieurs tissus d'épaisseur $x_i$ et de coefficient d'atténuation $\mu_i$ se calcule à partir de l'intensité $I_0$ du rayon X (aussi appelé faisceau incident) en sortie de sa source Slat ou Spa et de l'intensité I de ce même rayon X telle que détectée sur un point unitaire ou pixel de l'image correspondante, selon la formule $I = I_0\,e^{-\sum \mu_i x_i}$.

Le logiciel exécuté par le calculateur, calcule à l'aide de la valeur d'épaisseur de tissus mous traversés par ce rayon X donné :

- d'une part une valeur d'atténuation de ce rayon X donné par l'épaisseur de tissus mous qu'il a traversée ; et
- d'autre part une valeur d'atténuation de ce rayon X donné par l'épaisseur de tissus osseux qu'il a traversée.

A l'aide de la valeur d'épaisseur de tissus osseux traversée par ce rayon X donné, de la valeur d'atténuation de ce rayon X donné par la seule épaisseur de tissus mous qu'il a traversée, et de la valeur d'atténuation globale de ce rayon X donné, le logiciel estime une valeur représentative de la densité minérale osseuse des tissus osseux V traversés par ce rayon X donné.

Il va maintenant être explicité en référence aux figures 1, 1a et 2, un exemple d'estimation de répartition de DMO pour une vertèbre V.

Sur la figure 1a, les tissus mous, c'est-à-dire les organes autres que les tissus durs du squelette, ont un coefficient d'atténuation de rayons X qui est sensiblement homogène.

Au contraire, dans l'exemple de la figure 2, il existe une hétérogénéité de tissus mous qui présente de coefficient d'atténuation différent du coefficient d'atténuation des autres tissus mous. Cet exemple permet d'expliquer comment le dispositif selon l'invention qui comprend un calculateur et le logiciel décrit ci-avant met en oeuvre le procédé selon l'invention de matière automatisée.

**Rappel de l'équation d'atténuation des rayons X :**

Soit $I_0$ l'intensité du faisceau incident émis par la source, I l'intensité du faisceau / rayon telle que détectée sur un pixel de l'image, $\mu$ le coefficient d'atténuation de la matière traversée par le faisceau, et x l'épaisseur traversée de cette matière.

$$I = I_0 e^{-\mu x} \qquad\qquad 1$$

Si l'on a plusieurs milieux $\mu_i$ de plusieurs épaisseurs différentes $x_i$, alors l'équation d'atténuation est :

$$\frac{I}{I_0} = e^{-\sum \mu_i x_i} \qquad\qquad 2$$

Exemple d'utilisation des formules 1 et 2 pour l'estimation de la densité minérale osseuse d'une vertèbre (figure 1) :

Soit une vertèbre V donnée avec deux sources biplanes permettant d'avoir des images RX postéroantérieure PA et latérale Lat, et un rayon incident passant par cette vertèbre sur la vue Lat, et soient :

L1 et L2 les épaisseurs de tissus mous traversées de part et d'autre de la vertèbre V,

- $\mu_m$ le coefficient d'atténuation des tissus mous (qui peut être différencié lui-même en plusieurs coefficients, par exemple $\mu_{m1}$ et $\mu_{m2}$)
- $\mu_{vi}$ les différents coefficients d'atténuation dans la vertèbre (milieu hétérogène) au niveau de la ligne définissant la trajectoire du rayon incident dans la vertèbre, avec $x_{vi}$ les épaisseurs traversées dans cette ligne

Nous avons alors :

$$\frac{I}{I_0} = e^{-\mu_m L_1 - \mu_m L_2 - \sum \mu_{vi} x_{vi}} \qquad\qquad 3$$

Et donc

$$\frac{I}{I_0} = e^{-\mu_m(L_1+L_2)-\sum \mu_{vi}x_{vi}} \qquad\qquad 4$$

Et donc

$$\frac{I}{I_0 e^{-\mu_m(L_1+L_2)}} = e^{-\sum \mu_{vi}x_{vi}} \qquad\qquad 5$$

Et donc nous pouvons reconstruire l'image « DMO surfacique», qui ne comporte que le signal os, et qui est obtenue pour chaque rayon incident (et donc chaque pixel) en considérant le terme I corrigé (Icorr)

$$\mathrm{I_{corr}} = \frac{I}{I_0 e^{-\mu_m(L_1+L_2)}}$$

Avec cette modification du signal, nous pouvons considérer la démarche connue d'absorptiométrie par rayon X à simple énergie (Single-energy X-ray absorptiometry SXA), décrite par exemple dans la publication de Mabilleau G et al pour un os isolé (Mabilleau G, Mieczkowska A, Libouban H, Simon Y, Audran M, Chappard D. Comparison between quantitative X-ray imaging, dual energy X-ray absorptiometry and microCT in the assessment of bone mineral density in disuse-induced bone loss. J Musculoskelet Neuronal Interact. 2015 Mar;15(1):42-52).

Une calibration peut être faite avec un fantôme de calibration (éprouvette), généralement en lucite (dont le coefficient d'atténuation est proche des tissus mous) et aluminium (dont le coefficient d'atténuation est proche de l'os), puis le signal est redistribué entre cette plage de valeurs pour rehausser le signal os, ce qui permet au final d'avoir la DMO surfacique, et ceci avec une image en simple énergie.

Remarques :

**1) Calibration** : Pour la calibration (figure la ou 2), nous pouvons prendre avantage du fait que nous avons des zones qui ne sont constituées que de tissus mous et pour lesquelles nous connaissons la longueur L d'épaisseur traversée, donc nous pouvons avoir directement le coefficient d'atténuation des tissus mous du sujet considéré

**2) Rehaussement de contraste « os »** : cette image « os », lorsque la calibration a permis d'éliminer le signal tissus mous et de redistribuer le signal sur la plage entre tissus mous et os, permet de rehausser le contraste et peut donc être utile à la reconstruction 3D

**3) Présence d'hétérogénéités :** Du fait des variations interindividuelles et de spécificités au moment de la prise de radiographies, nous pouvons avoir des inhomogénéités locales de coefficient d'atténuation, qui perturbent le signal. Si nous avons des radiographies biplanes calibrées (figure 2), il est possible d'estimer sur les radiographies (postéoantérieure et latérale) par exemple, la dimension de l'élément hétérogène (noté avec l'indice h), et corriger son effet de la manière suivante :

◦ 1/ Estimation de Lpah et Llath (à partir des radiographies biplanes calibrées et d'un modèle géométrique de l'hétérogénéité)

◦ 2/ sur la vue postéroantérieure, pour les rayons Rpa1 qui traversent les tissus mous sans hétérogénéité, connaissant les épaisseurs traversées, on peut en déduire $\mu_m$ (coefficient d'atténuation des tissus mous)

◦ 3/ pour un rayon Rpa2 qui traverse l'hétérogénéité sur une longueur totale Lpa2 dont Llath du milieu h, le signal sur l'image résulte de $\mu_m$ ($L_{pa}$-$L_{lath}$) et $\mu_h$ $L_{lath}$, ce qui permet d'en déduire $\mu_h$.

◦ 4/ pour produire l'image de DMO en vue latérale, nous connaissons l'épaisseur globale traversée « hors os » (L1 + L2), l'épaisseur traversée par l'hétérogénéité $L_{pah}$ (grâce à la vue PA), les coefficients d'atténuation $\mu_m$ et $\mu_h$, et donc en reprenant l'équation 2 et le raisonnement déployé dans les équations 3 à 6 nous arrivons à l'image DMO définie par le terme :

$$I_{corr} = \frac{I}{I_0 e^{-\mu m\left(L_1 + L_2 - L_{pah}\right) - \mu_h\left(L_{pah}\right)}}$$

La valeur de $I_{corr}$ permet d'obtenir une valeur dite de DMO surfacique, telle qu'elle est obtenue en général à partir des modalités d'imagerie de référence (DXA correspondant à la méthode d'imagerie par absorptiométrie biphotonique à *rayons X*), cette valeur de DMO surfacique ne prend pas en compte l'hétérogénéité du tissu vertébral traversé.

Il est également possible, à partir de cette image de DMO surfacique, d'estimer une distribution de densité volumique selon la méthode décrite par Travert (thèse de sciences disponible sur https://tel.archives-ouvertes.fr/file/index/docid/834740/filename/TRAVERT.pdf, dont le titre est « Estimation du risque de fracture ostéoporotique du rachis thoraco-lombaire par un modèle en élément finis personnalisé » soutenue à l'ENSAM en 2012 : Brièvement, il s'agit de positionner une vertèbre comportant une distribution générique de densité minérale osseuse, et d'ajuster cette distribution à partir de la DMO surfacique données sur l'une des vues, ou de manière itérative à partir des données de DMO surfacique obtenues sur les deux vues.

On peut ainsi, à l'aide du logiciel / programme exécuté par un calculateur et analysant les première et deuxième images générées par projections de rayons X, estimer des positions de croisement de rayons dans l'espace, chaque position de croisement caractérisant le croisement entre un rayon donné provenant de la première source et un autre rayon donné provenant de la deuxième source. On peut associer des valeurs de densité minérale osseuse volumique à certains au moins des points de croisement.

## Revendications

1. Procédé d'estimation de la répartition de la densité minérale osseuse dans au moins une partie de squelette d'un individu, ce squelette étant composé de tissus osseux, ces tissus osseux étant entourés de tissus mous eux même entourés par une enveloppe externe de l'individu, le procédé étant **caractérisé en ce qu'**il comprend la génération :

   - d'une représentation géométrique en trois dimensions de ladite partie du squelette de l'individu; et
   - d'une représentation géométrique en trois dimensions d'au moins une portion de l'enveloppe externe (E) contenant ladite partie du squelette de l'individu ;
   - d'au moins une première image radiologique de ladite au moins une partie du squelette, cette première image étant générée par observation d'une projection de rayons X, provenant d'une première source, sur une première surface de détection, cette première image étant générée par projection de rayons X et représentant l'individu observé suivant une première incidence d'observation ;
   - d'au moins une deuxième image radiologique de ladite au moins une partie de squelette, cette deuxième image étant générée par projection de rayons X et représentant l'individu observé suivant une deuxième incidence d'observation distincte de la première incidence d'observation; puis
   à l'aide d'un logiciel prédéterminé, et pour plusieurs rayons X donnés parmi lesdits rayons X projetés, on estime pour chaque rayon X donné :

      - une valeur d'épaisseur de tissus mous traversés par ce rayon X donné ;
      - une valeur d'épaisseur de tissus osseux traversés par ce rayon X donné ; et
      - une valeur d'atténuation globale de ce rayon X donné, entre sa source et sa projection sur la surface de détection où il est projeté ; et

   à l'aide de la valeur d'épaisseur de tissus mous traversés par ce rayon X donné, et d'une estimée d'un coefficient d'atténuation des tissus mous traversés par ce rayon X donné, on déduit d'une part une valeur d'atténuation de ce rayon X donné par l'épaisseur de tissus mous qu'il a traversée et d'autre part une valeur d'atténuation de ce rayon X donné par l'épaisseur de tissus osseux qu'il a traversée ; puis
   à l'aide de la valeur d'épaisseur de tissus osseux traversée par ce rayon X donné, de la valeur d'atténuation de ce rayon X donné par l'épaisseur de tissus osseux qu'il a traversée et de la valeur d'atténuation globale de ce rayon X donné, le logiciel estime une valeur représentative de la densité minérale osseuse des tissus osseux traversés par ce rayon X donné.

2. Procédé d'estimation selon la revendication 1, dans lequel ladite deuxième image radiologique de ladite au moins une partie de squelette est générée par observation d'une projection de rayons X, provenant d'une deuxième source distincte et éloignée de la première source, sur une deuxième surface de détection.

**3.** Procédé d'estimation selon la revendication 2, dans lequel les première et deuxième surfaces de détection sont respectivement planes et sont orientées entre elles d'un angle de 90° à plus ou moins 60° et préférentiellement à plus ou moins 5° d'angle.

**4.** Procédé d'estimation selon l'une quelconque des revendications 1 à 3, dans lequel pour déduire ladite valeur d'atténuation dudit rayon X donné, par l'épaisseur de tissus mous qu'il a traversée, le logiciel estime un coefficient d'atténuation des tissus mous de l'individu.

**5.** Procédé d'estimation selon la revendication 4, dans lequel pour estimer le coefficient d'atténuation des tissus mous de l'individu, le logiciel recherche un rayon X particulier traversant uniquement des tissus mous de l'individu sans traverser de tissus osseux et à partir de la position de ce rayon X particulier et de la représentation géométrique en trois dimensions de la portion de l'enveloppe externe (E), le logiciel estime la valeur d'épaisseur de tissus mous traversés par ce rayon X particulier, et par observation d'un point de projection de ce rayon X particulier, le logiciel détermine une valeur d'atténuation subie par ce rayon X particulier lors de sa traversée des seuls tissus mous de l'individu, puis connaissant ladite valeur d'épaisseur de tissus mous traversés par ce rayon X particulier et ladite valeur d'atténuation subie par ce rayon X particulier, le logiciel calcule le coefficient d'atténuation associé aux seuls tissus mous de l'individu.

**6.** Procédé d'estimation selon la revendication 5, dans lequel connaissant :

- le coefficient d'atténuation des tissus mous de l'individu ;
- l'épaisseur de tissus mous traversée par ce rayon X donné ;
- une intensité ($I_0$) de ce rayon donné à sa source ; et
- une intensité (I) de ce rayon donné telle que détectée sur la surface de détection (P1, P2) sur laquelle il est projeté, le logiciel calcule :
- d'une part une valeur d'atténuation de ce rayon X donné par l'épaisseur de tissus mous qu'il a traversée; et
- d'autre part, une valeur d'atténuation globale de ce rayon X donné, entre sa source et la surface de détection sur laquelle il est projeté ; puis
- en corrigeant cette valeur d'atténuation globale de ce rayon X donné à l'aide de ladite valeur d'atténuation de ce rayon X donné par l'épaisseur de tissus mous qu'il a traversée, le logiciel calcule la valeur d'atténuation de ce rayon X donné par l'épaisseur de tissus osseux qu'il a traversée.

**7.** Procédé selon l'une quelconque des revendications 1 à 6, dans lequel ladite valeur représentative de la densité minérale osseuse des tissus osseux traversés par ce rayon X donné est un coefficient d'atténuation du tissu osseux traversé par ce rayon x donné.

**8.** Procédé selon l'une quelconque des revendications 1 à 7, dans lequel lesdites première et deuxième images correspondent respectivement à un même instant de détection des rayons X projetés sur les première et deuxième surfaces de détection.

**9.** Procédé selon l'une quelconque des revendications 1 à 7, dans lequel lesdites première et deuxième images correspondent respectivement à deux instants distincts de détection des rayons X projetés sur les première et deuxième surfaces de détection.

**10.** Procédé selon l'une quelconque des revendications 1 à 9, dans lequel les première et deuxième images illustrent des projections de l'individu en position debout.

**11.** Procédé selon l'une quelconque des revendications 1 à 10, dans lequel ladite représentation géométrique en trois dimensions de ladite au moins une portion de l'enveloppe externe contenant ladite partie du squelette de l'individu est déduite à partir desdites première et deuxième images radiologiques.

**12.** Procédé selon l'une quelconque des revendications 1 à 11, dans lequel ladite représentation géométrique en trois dimensions de ladite au moins une portion de l'enveloppe externe contenant ladite partie du squelette de l'individu est déduite à l'aide d'une observation de l'enveloppe réalisée à l'aide de moyens de capture optique.

**13.** Dispositif comportant:

- un calculateur ;

- des moyens de capture optique liés au calculateur pour observer une enveloppe externe (E) d'un individu ;
- des moyens de génération d'au moins une première image radiologique d'au moins une partie de squelette de l'individu par observation, suivant une première incidence d'observation, d'une projection de rayons X provenant d'une première source sur une première surface de détection ;
- des moyens de génération d'au moins une deuxième image radiologique de ladite au moins une partie de squelette par projection de rayons X suivant une deuxième incidence d'observation distincte de la première incidence d'observation,

**caractérisé en ce que**
le dispositif comporte un logiciel adapté pour réaliser des calculs ou des estimations ou déductions nécessaires à la mise en oeuvre du procédé d'estimation de la répartition de la densité minérale osseuse selon l'une quelconque des revendications 1 à 12.

## Patentansprüche

1. Schätzverfahren zum Schätzen der Verteilung der Knochenmineraldichte in mindestens einem Teil eines Skeletts einer Einzelperson, wobei dieses Skelett aus Knochengewebe besteht, wobei diese Knochengewebe von Weichgeweben umgeben sind, die selbst von einer äußeren Hülle der Einzelperson umgeben sind, wobei das Verfahren **dadurch gekennzeichnet ist, dass** es umfasst das Erzeugen:

   - einer dreidimensionalen geometrischen Darstellung des genannten Teils des Skeletts der Einzelperson; und
   - einer dreidimensionalen geometrischen Darstellung mindestens eines Abschnittes der äußeren Hülle (E), die den genannten Teil des Skeletts der Einzelperson enthält;
   - mindestens eines ersten Röntgenbildes des genannten mindestens einen Teil des Skeletts, wobei dieses erste Bild durch Beobachtung einer Projektion von Röntgenstrahlen, die aus einer ersten Quelle stammen, auf eine erste Erfassungsfläche erzeugt wird, wobei dieses erste Bild durch Projektion von Röntgenstrahlen erzeugt wird und die beobachtete Einzelperson gemäß einem ersten Beobachtungseinfallswinkel darstellt;
   - mindestens eines zweiten Röntgenbildes des genannten mindestens einen Teils des Skeletts, wobei dieses zweite Bild durch Projektion von Röntgenstrahlen erzeugt wird und die beobachtete Einzelperson gemäß einem zweiten Beobachtungseinfallswinkel darstellt, der sich von dem ersten Beobachtungseinfallswinkel unterscheidet; und dann mit Hilfe einer vorgegebenen Software und für mehrere gegebene Röntgenstrahlen unter den genannten projizierten Röntgenstrahlen für jeden gegebenen Röntgenstrahl geschätzt wird:
   - ein Wert für die Dicke der Weichgewebe, die von diesem gegebenen Röntgenstrahl durchlaufen werden;
   - ein Wert für die Dicke der Knochengewebe, die von diesem gegebenen Röntgenstrahl durchlaufen werden; und
   - ein Wert für die Gesamtdämpfung dieses gegebenen Röntgenstrahls zwischen seiner Quelle und seiner Projektion auf die Erfassungsfläche, auf die er projiziert wird; und

   mit Hilfe des Wertes für die Dicke der Weichgewebe, die von diesem gegebenen Röntgenstrahl durchlaufen werden, und eines Schätzwertes eines Dämpfungskoeffizienten der Weichgewebe, die von diesem gegebenen Röntgenstrahl durchlaufen werden, einerseits ein Wert für die Dämpfung dieses gegebenen Röntgenstrahls durch die Dicke der Weichgewebe, die er durchlaufen hat, und andererseits ein Wert für die Dämpfung dieses gegebenen Röntgenstrahls durch die Dicke der Knochengewebe, die er durchlaufen hat, hergeleitet wird; und dann mit Hilfe des Wertes für die Dicke der Knochengewebe, die von diesem gegebenen Röntgenstrahl durchlaufen werden, des Wertes für die Dämpfung dieses gegebenen Röntgenstrahls durch die Dicke der Knochengewebe, die er durchlaufen hat, und des Gesamtdämpfungswertes für diesen gegebenen Röntgenstrahl die Software einen Wert schätzt, der repräsentativ für die Knochenmineraldichte der von diesem gegebenen Röntgenstrahl durchlaufenen Knochengewebe ist.

2. Schätzverfahren nach Anspruch 1, bei dem das genannte zweite Röntgenbild des genannten mindestens einen Teils des Skeletts durch Beobachtung einer Projektion von Röntgenstrahlen, die aus einer zweiten Quelle stammen, die verschieden und entfernt zur ersten Quelle ist, auf eine zweite Erfassungsfläche erzeugt wird.

3. Schätzverfahren nach Anspruch 2, bei dem die erste und die zweite Erfassungsfläche jeweils eben und zueinander um einen Winkel von 90° plus oder minus einem Winkel von 60° und vorzugsweise plus oder minus einem Winkel von 5° ausgerichtet sind.

4. Schätzverfahren nach einem der Ansprüche 1 bis 3, bei dem die Software zum Herleiten des genannten Dämpfungswertes des genannten gegebenen Röntgenstrahls durch die Dicke der Weichgewebe, die er durchlaufen hat, einen Dämpfungskoeffizienten für die Weichgewebe der Einzelperson schätzt.

5. Schätzverfahren nach Anspruch 4, bei dem die Software zum Schätzen des Dämpfungskoeffizienten für die Weichgewebe der Einzelperson einen besonderen Röntgenstrahl sucht, der einzig die Weichgewebe der Einzelperson durchläuft, ohne die Knochengewebe zu durchlaufen, und anhand der Position dieses besonderen Röntgenstrahls und der dreidimensionalen geometrischen Darstellung des Abschnittes der äußeren Hülle (E) die Software den Wert für die Dicke der Weichgewebe schätzt, die von diesem besonderen Röntgenstrahl durchlaufen werden, und durch Beobachtung eines Projektionspunktes dieses besonderen Röntgenstrahls die Software einen Wert für die Dämpfung bestimmt, die von diesem besonderen Röntgenstrahl während seines Durchlaufens einzig der Weichgewebe der Einzelperson erfahren wird, und dann in Kenntnis dieses genannten Wertes für die Dicke der Weichgewebe, die von diesem besonderen Röntgenstrahl durchlaufen werden, und des genannten Wertes für die Dämpfung, die von diesem besonderen Röntgenstrahl erfahren wird, die Software den Dämpfungskoeffizienten berechnet, der einzig mit den Weichgeweben der Einzelperson in Verbindung steht.

6. Schätzverfahren nach Anspruch 5, bei dem in Kenntnis:

- des Dämpfungskoeffizienten für die Weichgewebe der Einzelperson;
- der Dicke der von diesem gegebenen Röntgenstrahl durchlaufenen Weichgewebe;
- einer Intensität ($I_0$) dieses gegebenen Strahls an seiner Quelle; und
- einer Intensität (I) dieses gegebenen Strahls, wie er auf der Erfassungsfläche (P1, P2), auf die er projiziert wird, erfasst wird, die Software berechnet:
- einerseits einen Wert für die Dämpfung dieses gegebenen Röntgenstrahls durch die Dicke der Weichgewebe, die er durchlaufen hat; und
- andererseits einen Gesamtdämpfungswert für diesen gegebenen Röntgenstrahl zwischen seiner Quelle und der Erfassungsfläche, auf die er projiziert wird; und dann
- durch Korrigieren dieses Gesamtdämpfungswerts für diesen gegebenen Röntgenstrahl mit Hilfe des genannten Wertes für die Dämpfung dieses gegebenen Röntgenstrahls durch die Dicke der Weichgewebe, die er durchlaufen hat, die Software den Wert für die Dämpfung dieses gegebenen Röntgenstrahls durch die Dicke der Knochengewebe berechnet, die er durchlaufen hat.

7. Verfahren nach einem der Ansprüche 1 bis 6, bei dem der genannte Wert, der repräsentativ für die Knochenmineraldichte der Knochengewebe ist, die von diesem Röntgenstrahl durchlaufen werden, ein Dämpfungskoeffizient des Knochengewebes ist, das von diesem gegebenen Röntgenstrahl durchlaufen wird.

8. Verfahren nach einem der Ansprüche 1 bis 7, bei dem das genannte erste und das genannte zweite Bild einem selben Zeitpunkt des Erfassens der Röntgenstrahlen entsprechen, die auf die erste bzw. die zweite Erfassungsfläche projiziert werden.

9. Verfahren nach einem der Ansprüche 1 bis 7, bei dem das genannte erste und das genannte zweite Bild zwei unterschiedlichen Zeitpunkten des Erfassens der Röntgenstrahlen entsprechen, die auf die erste bzw. die zweite Erfassungsfläche projiziert werden.

10. Verfahren nach einem der Ansprüche 1 bis 9, bei dem das erste und das zweite Bild Projektionen der Einzelperson in der aufrechten Position zeigen.

11. Verfahren nach einem der Ansprüche 1 bis 10, bei dem die genannte dreidimensionale geometrische Darstellung des genannten mindestens einen Abschnitts der äußeren Hülle, die den genannten Teil des Skeletts der Einzelperson enthält, anhand des genannten ersten und zweiten Röntgenbildes hergeleitet wird.

12. Verfahren nach einem der Ansprüche 1 bis 11, bei dem die genannte dreidimensionale geometrische Darstellung des genannten mindestens einen Abschnitts der äußeren Hülle, die den genannten Teil des Skeletts der Einzelperson enthält, mit Hilfe einer Beobachtung der Hülle hergeleitet wird, die mit Hilfe optischer Erfassungsmittel erzeugt wird.

13. Vorrichtung, umfassend:

- einen Rechner;
- optische Erfassungsmittel, die mit dem Rechner verbunden sind, um eine äußere Hülle (E) einer Einzelperson zu beobachten;

- Erzeugungsmittel zur Erzeugung mindestens eines ersten Röntgenbildes mindestens eines Teils des Skeletts der Einzelperson durch Beobachtung gemäß einem ersten Beobachtungseinfallswinkel einer Projektion von Röntgenstrahlen, die aus einer ersten Quelle stammen, auf eine erste Erfassungsfläche;
- Erzeugungsmittel zur Erzeugung mindestens eines zweiten Röntgenbildes des genannten mindestens einen Teils des Skeletts durch Projektion von Röntgenstrahlen gemäß einem zweiten Beobachtungseinfallswinkels, der sich von dem ersten Beobachtungseinfallswinkel unterscheidet,

**dadurch gekennzeichnet, dass**
die Vorrichtung eine Software umfasst, die geeignet ist, um Berechnungen oder Schätzungen oder Herleitungen durchzuführen, die zum Durchführen des Schätzverfahrens zum Schätzen der Verteilung der Knochenmineraldichte nach einem der Ansprüche 1 bis 12 erforderlich sind.

**Claims**

1. An estimation method for estimating the bone mineral density distribution in at least one portion of a person's skeleton, the skeleton being made up of bone tissue, the bone tissue being surrounded by soft tissue, in turn surrounded by an outline of the person, the method being **characterized in that** it comprises generating:

   - a geometrical representation in three dimensions of said portion of the person's skeleton;
   - a geometrical representation in three dimensions of at least one portion of the outline (E) containing said portion of the person's skeleton;
   - at least one first X-ray image of said at least one portion of the skeleton, this first image being generated by observing a projection onto a first detection surface of X-rays, coming from a first source, the first image being generated by projecting X-rays and representing the person as observed from a first observation angle of incidence; and
   - at least one second X-ray image of said at least one portion of the skeleton, the second image being generated by projecting X-rays and representing the person as observed from a second observation angle of incidence distinct from the first observation angle of incidence; and then

   using predetermined software and for a plurality of given X-rays selected from among said projected X-rays, estimating for each given X-ray:

   - a value for the thickness of soft tissue through which the given X-ray has passed;
   - a value for the thickness of bone tissue through which the given X-ray has passed; and
   - a value for the overall attenuation of the given X-ray between its source and its projection onto the detection surface onto which it is projected; and

       - using the thickness value for soft tissue through which the given X-ray passes, and using an estimate for an attenuation coefficient of the soft tissues through which the given X-ray passes, deducing firstly a value for the attenuation of the given X-ray due to the thickness of soft tissue through it has passed and secondly a value for the attenuation of the given X-ray due to the thickness of bone tissue through which it has passed; and then
       - using the value for the thickness of bone tissue through which the given X-ray has passed, the value for the attenuation of the given X-ray due to the thickness of bone tissue through which it has passed, and the overall value for attenuation of the given X-ray, the software estimates a value representative of the bone mineral density of the bone tissue through which the given X-ray has passed.

2. An estimation method according to claim 1, wherein said second X-ray image of said at least one portion of the skeleton is generated by observing a projection onto a second detection surface of X-rays coming from a second source that is distinct and spaced apart from the first source.

3. An estimation method according to claim 2, wherein the first and second detection surfaces are respectively plane and oriented relative to each other at an angle of 90° plus or minus 60°, and preferably plus or minus 5° of angle.

4. An estimation method according to any one of claims 1 to 3, wherein in order to deduce said value for the attenuation of said given X-ray due to the thickness of soft tissue through which it has passed, the software estimates an attenuation coefficient for the soft tissues of the person.

14

**5.** An estimation method according to claim 4, wherein in order to estimate the attenuation coefficients of the soft tissues of the person, the software looks for a particular X-ray that passes solely through soft tissues of the person without passing through bone tissues, and from the position of this particular X-ray and from the geometrical representation in three dimensions of the portion of the outline (E), the software estimates the value of the thickness of soft tissues through which this particular X-ray passes, and by observing a projection point of this particular X-ray, the software determines a value for the attenuation to which the particular X-ray is subjected on passing solely through soft tissues of the person, and then knowing said thickness value for the soft tissues through which a particular X-ray passes and said value for the attenuation to which the particular X-ray is subjected, the software calculates the attenuation coefficient associated solely with the person's soft tissues.

**6.** An estimation method according to claim 5, wherein, knowing:

- the attenuation coefficient of the person's soft tissue;
- the thickness of the soft tissue through which the given X-ray passes;
- the intensity ($I_0$) of the given ray at its source; and
- the intensity (I) of the given ray as detected on the detection surface (P1, P2) onto which it is projected;

the software calculates:

- firstly a value for the attenuation of the given X-ray due to the thickness of the soft tissue through which it has passed; and
- secondly, a value for the overall attenuation of the given X-ray, between its source and the detection surface onto which it is projected; and then
- by correcting this overall attenuation value of the given X-ray by using said attenuation value of the given X-ray due to the thickness of soft tissue through which it has passed, the software calculates the value of the attenuation of the given X-ray due to the thickness of bone tissue through which it has passed.

**7.** A method according to any one of claims 1 to 6, wherein said value representative of the bone mineral density of bone tissue through which the given X-ray passes is an attenuation coefficient of the bone tissue through which the given X-ray passes.

**8.** A method according to any one of claims 1 to 7, wherein said first and second images correspond respectively to detecting X-rays projected onto the first and second detection surfaces at a common instant.

**9.** A method according to any one of claims 1 to 7, wherein said first and second images correspond respectively to detecting X-rays projected onto said first and second detection surfaces at two distinct instants.

**10.** A method according to any one of claims 1 to 9, wherein the first and second images illustrate projections of the person in the standing position.

**11.** A method according to any one of claims 1 to 10, wherein said geometrical representation in three dimensions of said at least one portion of the outline containing said portion of the person's skeleton is deduced from said first and second X-ray images.

**12.** A method according to any one of claims 1 to 11, wherein said geometrical representation in three dimensions of said at least one portion of the outline containing said portion of the person's skeleton is deduced using an observation of the outline made using optical capture means.

**13.** A device including:

- a computer;
- optical capture means linked to the computer for observing an outline (E) of a person;
- means for generating at least one first X-ray image of at least one portion of the skeleton of the person by observing, from a first observation angle of incidence, a projection of X-rays coming from a first source onto a first detection surface;
- means for generating at least one second X-ray image of said at least one portion of the skeleton, by projection according to a second observation angle of incidence distinct from said first observation angle of incidence, **characterized in that** the device comprise a software adapted to perform calculations or estimations or deduc-

15

tions necessary to implement the estimation method for estimating the bone mineral density distribution according to any one of claims 1 to 12.

Fig 1

Fig 1a

Fig 2

**EP 3 291 733 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20060204069 A1
- FR 2856170
- WO 2008146069 A

**Littérature non-brevet citée dans la description**

- **HUMBERT L ; DE GUISE JA ; AUBERT B ; GODBOUT B ; SKALLI W.** 3D reconstruction of the spine from biplanar X-rays using parametric models based on transversal and longitudinal inferences. *Med Eng Phys.,* Juillet 2009, vol. 31 (6), 681-7
- **DE RAPHAEL DUMAS ; RACHID AISSAOUI ; MEMBER, IEEE ; DAVID MITTON ; WAFA SKALLI ; JACQUES A.** Personalized Body Segment Parameters From Biplanar Low-Dose Radiography. *de Guise publiée au journal IEEE TRANSACTIONS ON BIOMEDICAL ENGINEERING,* Octobre 2005, vol. 52 (10
- Subject-specific body segment parameters estimation using biplanar X-rays: a feasibility study. **DE BAPTISTE SANDOZ ; SÉBASTIEN LAPORTE ; WAFA SKALLI ; DAVID MITTON.** Computer Methods in Biomechanics and Biomédical Engineering. Taylor & Francis, Décembre 2010, vol. 13, 649-654
- **MABILLEAU G ; MIECZKOWSKA A ; LIBOUBAN H ; SIMON Y ; AUDRAN M ; CHAPPARD D.** Comparison between quantitative X-ray imaging, dual energy X-ray absorptiometry and microCT in the assessment of bone mineral density in disuse-induced bone loss. *J Musculoskelet Neuronal Interact,* Mars 2015, vol. 15 (1), 42-52